# EUROPEAN PATENT APPLICATION

(11) **EP 4 667 016 A1**
(43) Date of publication of application: **24.12.2025**
(21) Application number: 24755900.8
(22) Date of filing: 19.01.2024
(51) Int. Cl.: A61K 41/00, A61P 35/00, A61P 35/02

(54) **BORON-CONTAINING DRUG AND PREPARATION METHOD THEREFOR**

(30) Priority: 17.02.2023 CN 202310131163
(71) Applicant: Chengdu New Radiomedicine Technology Co., Ltd., Chengdu, Sichuan 610200 (CN)
(72) Inventor: QIAN, Zhiyong, Chengdu, Sichuan 610000 (CN); DAI, Liqun, Chengdu, Sichuan 610213 (CN); BAI, Bing, Chengdu, Sichuan 610200 (CN); ZHAO, Xiaosheng, Ganzhou, Jiangxi 341000 (CN); ZHANG, Chixiang, Chengdu, Sichuan 610083 (CN); ZHANG, Jun, Guangyuan, Sichuan 628300 (CN); CAI, Jiming, Chengdu, Sichuan 610000 (CN)
(74) Representative: Barker Brettell LLP
(86) International application number: PCT/CN2024/073245
(87) International publication number: WO 2024/169525

(57) **Abstract**

The present invention relates to a boron-containing drug and a preparation method thereof. The boron-containing drug is obtained by means of polymerizing dopamine and a derivative thereof with a boron-containing compound. The concentration of the boron-containing drug in tumors can reach up to 70 µg/g. Furthermore, the boron concentration ratio of tumors to blood is significantly increased and can reach 70:1. Moreover, the boron-containing drug can be quickly cleared from blood and normal organs, drug dosage can be significantly decreased, side effects can be reduced, and tumor treatment effects can be significantly enhanced.

## Description

### TECHNICAL FIELD

The present invention belongs to the technical field of tumor therapeutic drugs, in particular to a boron-containing drug and a preparation method thereof.

### BACKGROUND

Currently, two kinds of boron-containing drugs have been used in clinical practice, namely, borylphenylalanine (BPA) and sodium mercaptoundecahydrocloso-dodecaborate (BSH). At present, a small number of clinical trials have been conducted on BSH-based drugs, but no formal marketing approval has been obtained. BSH is favored by researchers due to its high content of boron-10, but the difficulty in the synthesis and purification processes of the active pharmaceutical ingredient leads to limited availability and high cost. Additionally, the clinical safety and efficacy of BSH have not been fully certified, and research based on BSH is currently decreasing. BPA has received more attention in recent years because of its relatively simple synthesis and purification processes, good compound stability, and more comprehensive research and certification of its clinical safety and efficacy.

Currently, BPA-based drugs are mainly made into solution preparations or freeze-dried powder with BPA as the active pharmaceutical ingredient. Its main problem is the low tumor uptake rate and rapid blood clearance, which require a large clinical dosage of the drugs. At present, the main challenge for boron-containing drugs is how to improve the targeting and utilization of tumors.

In clinical neutron capture therapy for tumor treatment, the concentration of ¹⁰B in tumor tissue needs to be maintained at about 30µg/g during neutron irradiation to achieve a good therapeutic effect. The tumor/normal tissue concentration ratio (T/N ratio) of BPA can only reach 3: 1. When the BPA level in cancer cells increases, the BPA will be excreted through a "reverse transport" mechanism. The clinically approved BPA-based solution preparation, Steboronine^{®}, in order to maintain the local ¹⁰B concentration in tumors during treatment, requires adult patients to receive an intravenous infusion of borophalan (10B) at a rate of 200 mg/kg per hour for 2 hours before treatment, and at a rate of 100 mg/kg per hour for 30-60 minutes during treatment. Due to its poor tumor targeting or low T/N ratio and rapid metabolism, a 60kg patient needs a high clinical dosage of 36g BPA for one treatment, which not only increases the cost of treatment, but also increases the risk of safety. Furthermore, the poor tumor targeting or low T/N ratio requires higher precision of neutron source irradiation in clinical applications, while also increasing the risk of normal tissue receiving radiation dose exceeding the standard (if normal tissue is irradiated, due to the low T/N ratio, the normal tissue will have a higher boron content and is also prone to damage). Moreover, this limits the clinical application and expansion of indications for the drug.

In view of this, the present invention is presented.

### SUMMARY

To address the technical problem in the background, an object of the present invention is to develop a safe and effective boron-containing drug preparation with better tumor targeting and higher utilization rate, based on dopamine and its derivative and a boron-containing compound. The invention also aims to develop a preparation method of the boron-containing drug and study its potential application in the treatment of tumors and other diseases. The boron-containing drug preparation can significantly reduce the dosage of boron-containing drug on the basis of safety and efficacy, and thereby expanding more indications on the basis of good targeted distribution of tumors.

To achieve the above purpose, the first technical solution adopted by the present invention is:
A boron-containing drug which is obtained by polymerizing dopamine and its derivative with a boron-containing compound.

Preferably, the boron-containing compound includes, but is not limited to, any one or more of boric acid, phenylalanine boric acid and its derivative, proline boric acid, aspartic acid boric acid, tyrosine boric acid, cysteine boric acid, methionine boric acid, serine boric acid, 5-amino-2,3-difluorophenyl boric acid, 3-amino-5-cyanophenyl boric acid, 3-amino-4-methylphenyl boric acid, 3-amino-phenylboric acid hydrochloride, and 4-carbamoyl phenylboric acid.

Preferably, the boron-containing compound comprises any one of the phenylalanine boric acid and its derivative.

Preferably, the phenylalanine boric acid and its derivative are borylphenylalanine (BPA).

Preferably, the dopamine and its derivative comprise any one or more of dopamine, 4-(2-aminopropyl)phenyl-1,2-diol, norepinephrine (1-(3,4-hydroxyphenyl)-2-amino ethanol), L-methyldopa (2-methyl-3-(3,4-dihydroxyphenyl)-L-alanine), droxidopa ((2S,3R)-(2-amino-3-(3,4-dihydroxyphenyl)-3-hydroxy acrylic acid), and 5-hydroxydopamine; preferably, the dopamine and its derivative are dopamine, L-methyldopa (2-methyl-3-(3,4-dihydroxyphenyl)-L-alanine) or 5-hydroxydopamine.

Preferably, the weight ratio of the dopamine and its derivative to the boron-containing compound is (1-10) : 1.

Preferably, the weight ratio of the dopamine and its derivative to the boron-containing compound is (3-4) : 1.

Preferably, the boron content in the boron-containing drug is 0.1% to 1.0%.

Preferably, an indication for the boron-containing drug comprises diseases such as head and neck tumors, lung cancer, peritoneal cancer, hepatocellular cancer, gastric cancer, melanoma, pancreatic cancer, brain glioma, cervical cancer, ovarian cancer, liver cancer, bladder cancer, breast cancer, colon cancer, colorectal cancer, uterine cancer, salivary gland cancer, kidney cancer, prostate cancer, vulvar cancer, thyroid cancer, B cell lymphoma or leukemia; Preferably, the indication is head and neck tumors, melanoma or brain glioma.

The second technical solution adopted by the present invention is:
A method for preparing a boron-containing drug, comprising steps of:
mixing and reacting an aqueous solution of dopamine and its derivative with an aqueous solution of a boron-containing compound, separating a solid from a liquid to obtain the solid, and washing and freeze-drying the solid.

Preferably, the mixing and reacting is performed at pH from 6 to 14.

Preferably, the mixing and reacting is performed at pH from 8 to 11.

Preferably, a time of the mixing and reacting is 0.5 hour to 48 hours, and a stirring speed during the reacting is over 200 RPM.

Compared to the prior art, the present invention has the following beneficial effects:
1. The boron-containing drug prepared by the present invention can reach a concentration of up to 70 µg/g in tumors, and the boron concentration ratio of tumor to blood is significantly increased and can reach 70:1, which is much higher than the tumor/normal tissue boron concentration ratio of current BPA drug preparations. It greatly enhances the tumor uptake rate, significantly improves the enrichment of boron in tumors, enhances the therapeutic efficacy, and can be quickly cleared from blood and normal organs, significantly reducing the drug dosage and side effects, thereby offering superior tumor targeting and higher utilization rate, and significantly improving the tumor treatment effects.
2. The drug of the present invention has the property of long retention time in tumor tissue, with a long treatment window, overcoming the drawback of the need for continuous infusion during the boron neutron irradiation in the prior art. The experiment of subcutaneous brain glioma in mice show that when the dosage is 150 mg/kg, the boron concentration can be maintained above 30 µg/g during the range of 24 hours to 36 hours, the treatment window is more than 12 hours, and there is no need for continuous infusion of the boron-containing drug during the treatment, making the treatment process easier to implement.
3. Ordinary nanomedicines cannot pass through the blood-brain barrier and are therefore usually not used for the treatment of brain tumors. However, the boron-containing drug of the present invention can pass through the blood-brain barrier and enrich in gliomas, and the drug can be maintained above therapeutic concentration for a longer time, making it possible to be used in the treatment of brain tumors.
4. The boron-containing drug of the present invention has low toxicity and good biocompatibility.
5. The boron-containing drug of the present invention has the property of heat generation under near-infrared light irradiation, i.e., photothermal properties. The photothermal conversion efficiency of the boron-containing drug is 39.46%, enabling combined boron neutron and photothermal therapy.

### DESCRIPTION OF THE DRAWINGS

Fig. 1 shows the Fourier transform infrared (FT-IR) spectrum of the boron-containing drug in Example 1;
Fig. 2 shows the particle size distribution diagram of the boron-containing drug in Example 1;
Fig. 3 shows the SEM diagram of the boron-containing drug in Example 2;
Fig. 4 shows the cell survival rates at 24h, 48h and 72h, obtained from the cytotoxicity detection of the boron-containing drug using three tumor cell lines with high sialic acid expression, HepG2, SKOV3, and H22 in example 3;
Fig. 5 shows the distribution map of boron in mice at 12h, 24h and 36h after drug administration, obtained from the in vivo distribution experiment of the boron-containing drug in a mouse subcutaneous brain glioma model in Example 4;
Fig. 6 shows the distribution map of boron in mice at 24 hours after drug administration, obtained from the in vivo distribution experiment of the boron-containing drug in a mouse orthotopic brain glioma model in Example 5, and.
Fig. 7 shows the SEM diagram of the boron-containing drug in Example 8;
Fig. 8 shows the temperature elevation curve of the boron-containing drug under the condition of 808 nm near-infrared laser irradiation at a power density of 1.5 W/cm² in Example 13.

### DETAILED DESCRIPTION

To better understand the technical solution of the present invention, the following description will be made in conjunction with the accompanying drawings and examples. The ways to implement the present invention include but are not limited to the following examples, which are provided to illustrate the invention but are not intended to limit the scope of the invention. Unless otherwise specified, the technical means used in the examples are conventional means known to those skilled in the art. The experimental methods in the following examples are conventional unless otherwise indicated.

The first embodiment of the present invention provides a boron-containing drug, which is obtained by polymerizing dopamine and its derivative with a boron-containing compound.

The boron-containing drug in this embodiment has a particle size of 50 to 1000 nanometers, and the drug can enter cells and pass through the blood-brain barrier.

This embodiment is mainly to improve the targeting property of the boron-containing drug in the body through optimization of composition and structure, increasing the T/N ratio of tumor to normal tissue, prolonging the retention time of the drug in the tumor, and allowing for rapid metabolism from the blood and normal tissues, thereby reducing the dosage of the boron-containing drug, lowering the risk of safety during treatment, and achieving better tumor targeting and higher utilization, significantly improving the therapeutic effect on tumors. This enhances the therapeutic effect of the boron-containing drug and expands its indications while significantly reducing the cost of drug treatment.

The boron-containing drug of this embodiment can enter cells via endocytosis, but cannot be expelled by tumor cells through reverse transport mechanisms, thus extending its retention time in tumor cells. This solves the issue of high BPA dosage in boron neutron capture therapy, further increasing the enrichment of the boron-containing drug at the tumor site and thereby improving the therapeutic effect. The distribution experiment of subcutaneous brain glioma in mice shows that when the dosage is 150 mg/kg, the boron concentration can be maintained above 30 µg/g during the range of 24 hours to 36 hours, the treatment window is more than 12 hours, and there is no need for continuous infusion of the boron-containing drug during the treatment, which brings great convenience to the treatment of patients.

The indication for the boron-containing drug of this embodiment includes, but is not limited to, diseases such as, head and neck tumors, lung cancer, peritoneal cancer, hepatocellular cancer, gastric cancer, melanoma, pancreatic cancer, brain glioma, cervical cancer, ovarian cancer, liver cancer, bladder cancer, breast cancer, colon cancer, colorectal cancer, uterine cancer, salivary gland cancer, kidney cancer, prostate cancer, vulvar cancer, thyroid cancer, B cell lymphoma or leukemia; preferably head and neck tumors, melanoma or brain glioma.

The boron-containing drug can be metabolized (biodegradable) in the body, and its concentration rapidly decreases after injection into the body for 48 hours until it is completely metabolized and cleared.

The boron-containing drug can generate heat under near-infrared light irradiation, having strong near-infrared absorption and high photothermal conversion efficiency. By irradiating 0.1mg/mL boron-containing drug with 808nm near-infrared light, the photothermal conversion efficiency is about 39%, enabling combined boron neutron therapy and photothermal therapy.

For the selection of raw materials, the boron-containing compound includes, but is not limited to, any one or more of boric acid, phenylalanine boric acid and its derivative, proline boric acid, aspartic acid boric acid, tyrosine boric acid, cysteine boric acid, methionine boric acid, serine boric acid, 5-amino-2,3-difluorophenyl boric acid, 3-amino-5-cyanophenyl boric acid, 3-amino-4-methylphenyl boric acid, 3-amino-phenylboric acid hydrochloride, and 4-carbamoyl phenylboric acid. Preferably, it includes any one of phenylalanine boric acid and its derivative; more preferably, borylphenylalanine (BPA).

The dopamine and its derivative comprise any one or more of dopamine, 4-(2-aminopropyl)phenyl-1,2-diol, norepinephrine (1-(3,4-hydroxyphenyl)-2-amino ethanol), L-methyldopa (2-methyl-3-(3,4-dihydroxyphenyl)-L-alanine), droxidopa ((2S,3R)-(2-amino-3-(3,4-dihydroxyphenyl)-3-hydroxy acrylic acid), and 5-hydroxydopamine; preferably, dopamine, L-methyldopa (2-methyl-3-(3,4-dihydroxyphenyl)-L-alanine) or 5-hydroxydopamine; preferably, the dopamine and its derivative are dopamine, L-methyldopa (2-methyl-3-(3,4-dihydroxyphenyl)-L-alanine), or 5-hydroxydopamine.

For the amount of the raw material components, the weight ratio of the dopamine and its derivative to the boron-containing compound is (1-10): 1, preferably (3-4):1.

The boron content in the boron-containing drug is 0.1% to 1.0%.

The second embodiment of the present invention provides a method for preparing a boron-containing drug, comprising steps of: mixing and reacting an aqueous solution of dopamine and its derivative with an aqueous solution of a boron-containing compound, separating a solid from a liquid to obtain the solid, and washing and freeze-drying the solid.

The mixing and reacting is performed at pH of 6-14, preferably 8-11. A time of the mixing and reacting is 0.5 hour to 48 hours, and a stirring speed during the reacting is over 200 RPM.

The solvent for the mixing and reacting is not particularly limited and the reacting can be carried out directly in an aqueous solution, or in solutions of methanol, ethanol, acetone, DMF, THF, or any mixture thereof.

The boron-containing drug of the present invention has good redispersibility after freeze-drying, and the redispersing solvents include but are not limited to one of sterile water for injection, sodium chloride injection, glucose injection, and PBS buffer solution.

The following examples illustrate the boron-containing drug and its properties.

### Example 1: Preparation of Boron-Containing Drug

(1) Weighing 80mg of dopamine hydrochloride into a reaction flask, adding 34 mL of 30% ethanol solution, and stirring to dissolve to obtain a solution (1);
(2) Weighing 20mg of BPA into a centrifuge tube, adding 3mL of water, and then adding 0.2mL of 2.5mol/L NaOH solution until BPA is completely dissolved to obtain a solution (2);
(3) Adding the solution (2) to the solution (1), adjusting the pH to about 10 with NaOH, and stirring at 300 RPM for 24 hours to obtain a solution (3);
(4) Centrifuging the solution (3) at 10000 RPM for 10 minutes and washing it with pure water three times to obtain a product; and
(5) Suspending the product in 6 mL of water and freeze-drying to obtain the boron-containing drug.

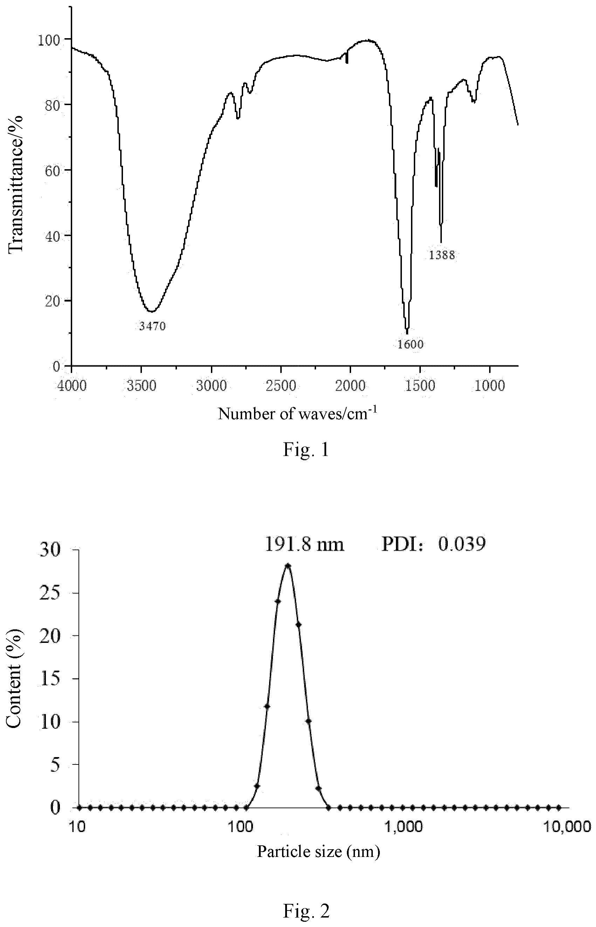

The infrared spectrum of the boron-containing drug prepared in this example is shown in Fig. 1, where the boron-containing drug has an infrared absorption peak for the B-N bond at 1388 cm⁻¹. The particle size distribution is shown in Fig. 2, with an average particle size of 191.8 nm.

### Example 2: Redispersibility Experiment of Boron-Containing Drug after Freeze-Drying

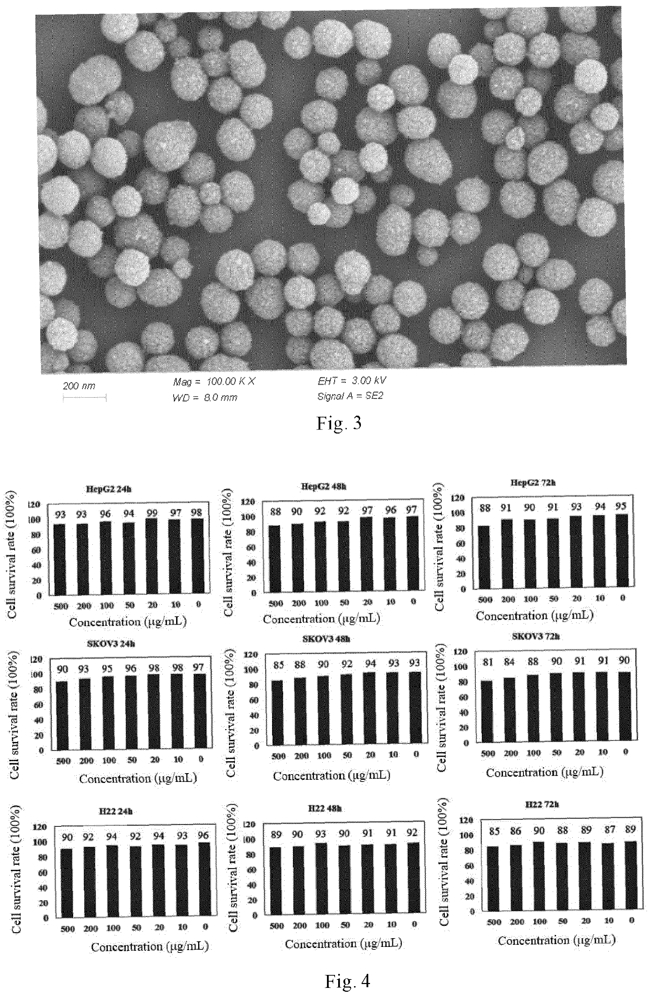

5mg of the boron-containing drug of Example 1 was taken and 5mL of solvent which includes but is not limited to any one of sterile water for injection, sodium chloride injection, glucose injection, PBS buffer, and water was added as a dispersing agent. After manual mixing, SEM detection was performed. The morphology is characterized as spherical as shown in Fig. 3.

### Example 3: In vitro Cytotoxicity Evaluation of Boron-Containing Drug over 24-72 hours

The evaluation time is 24-72 hours, and the detection method is the Cell Counting Kit-8 (CCK8), a rapid and highly sensitive assay kit for cell proliferation and cytotoxicity, which uses three tumor cell lines with high sialic acid expression, HepG2, SKOV3, and H22 for detection.

The specific operation steps are as follows: (1) Preparing cell suspensions of HepG2, SKOV3, and H22 cells in good growth condition at a certain concentration (5×10³ cells/100µL), respectively, and adding 100µL of the cell suspensions to each well of a 96-well cell culture plate;
(2) Adding 10µL of the boron-containing drug of Example 1 at concentrations of 0, 10, 20, 50, 100, 200, and 500µg/mL to the culture wells, respectively, and continuing to culture for a certain time in a 37°C incubator;
(3) Sampling at 24h, 48h, and 72h, respectively, and detecting the absorbance at 450nm to calculate the cell survival rate. The cell survival rate is above 80% at 72h, indicating that the cytotoxicity of the boron-containing drug is very low. The specific results are shown in Fig. 4.

### Example 4: In vivo Distribution Experiment of Boron-Containing Drug in a Mouse Subcutaneous Brain Glioma Model

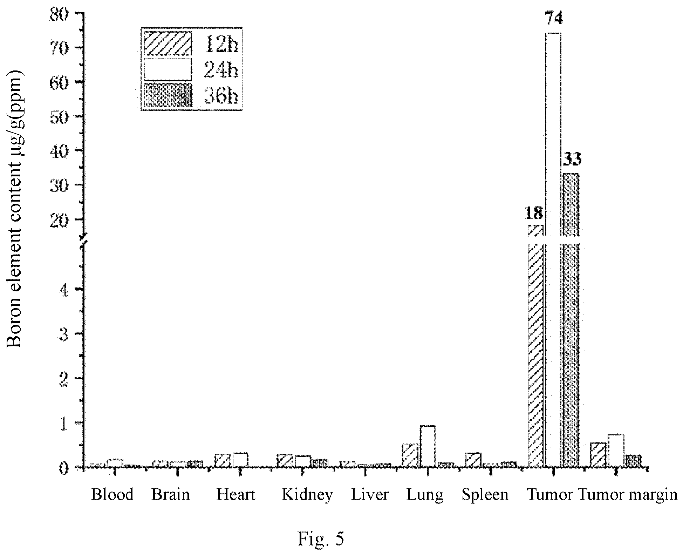

The boron-containing drug of Example 1 was taken and injected into tumor-bearing mice via the tail vein with a dosage of 150mg/kg. At 12h, 24h, and 36h after administration, the heart, liver, spleen, lung, kidney, blood, brain, tumor, and normal tissue at the tumor margin of the mice were collected, weighed, and digested. The boron concentration in each organ was measured using ICP-MS. At 24h, the boron concentration at the tumor site reaches 74µg/g, with a T/N ratio as high as 70:1. The distribution of boron in mice at 12h, 24h, and 36h after administration is shown in Fig. 5 and Table 1.

**Table 1: Distribution of Boron in Mice at 12h, 24h, and 36h after Administration**

| Sites | B element content at different times (µG/G) | | |
|---|---|---|---|
| | 12h | 24h | 36h |
| Heart | 0.08189 | 0.17047 | 0.04152 |
| Liver | 0.13816 | 0.11147 | 0.12833 |
| Spleen | 0.29927 | 0.31259 | 0.00946 |
| Lung | 0.29131 | 0.24431 | 0.16358 |
| Kidney | 0.12804 | 0.05531 | 0.07213 |
| Blood | 0.51842 | 0.92589 | 0.09688 |
| Brain | 0.31613 | 0.0797 | 0.10611 |
| Tumor | 18.1967 | 74.0081 | 33.2075 |
| Normal tissue at the tumor margin | 0.54357 | 0.73641 | 0.26313 |

This example investigates the distribution in subcutaneous brain glioma model animals, with injection of the boron-containing drug via tail vein at a dosage of 150mg/kg. At 12h, 24h, and 36h after administration, the heart, liver, spleen, lung, kidney, blood, brain, tumor, and normal tissue at the tumor margin of the mice were collected, and the boron concentration in each organ was measured using ICP-MS. At 24h, the boron concentration in the tumor reaches a maximum of 74µg/g, and remains above 30µg/g by 36h. The treatment window is more than 12 hours, and there is no need for continuous infusion of the boron-containing drug during the treatment. Moreover, the T/N ratio can reach up to 70:1 (Table 1), indicating minimal harm to normal tissue during boron neutron capture therapy.

**Example 5:** In vivo Distribution Experiment of Boron-Containing Drug in a Mouse Orthotopic Brain Glioma Model

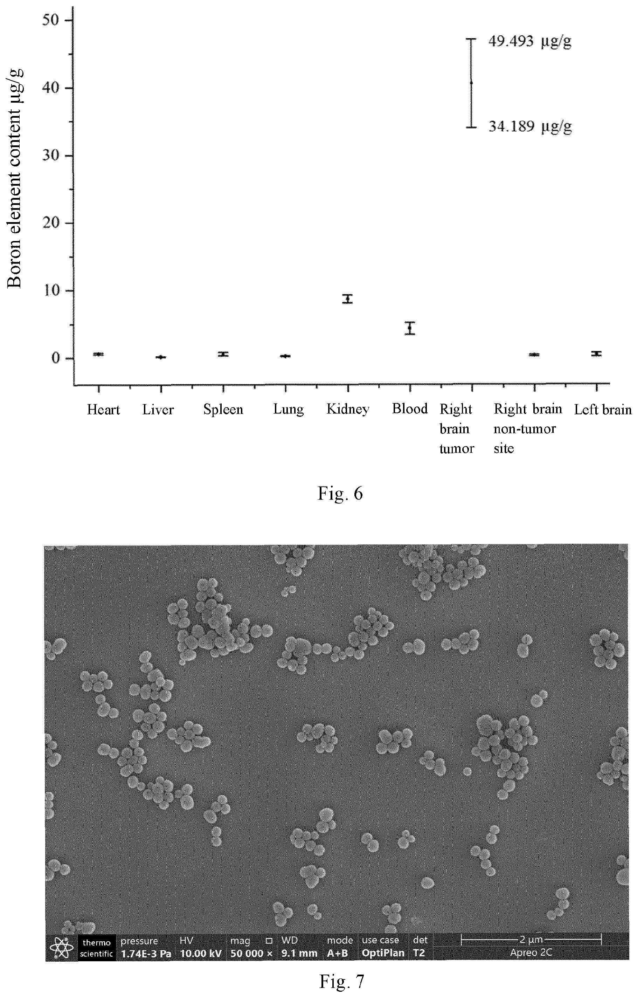

The boron-containing drug of Example 1 was taken and injected into tumor-bearing mice via the tail vein with a dosage of 100 mg/kg. At 24h after administration, the heart, liver, spleen, lung, kidney, blood, right brain tumor site, right brain non-tumor site, and left brain of the mice were collected, weighed, and digested. The boron concentration in each organ was measured using ICP-MS. The boron concentration at the tumor site is all above 30µg/g, with a T/N ratio greater than 7:1. The distribution of boron in mice at 24h after administration is shown in Fig. 6 and Table 2.

**Table 2: Distribution of Boron in Mice at 24h after Administration**

| Sites | Boron element content of mice with different Numbers (µg/g) | | |
|---|---|---|---|
| | Number 1 | Number 2 | Number 3 |
| Heart | 0.784 | 0.552 | 0.407 |
| Liver | 0.109 | 0.194 | 0.111 |
| Spleen | 0.937 | 0.343 | 0.359 |
| Lung | 0.230 | 0.226 | 0.115 |
| Kidney | 8.810 | 7.894 | 9.316 |
| Blood | 5.377 | 3.276 | 4.225 |
| Right brain tumor site | 37.772 | 34.189 | 49.493 |
| Right brain non-tumor site | 0.181 | 0.495 | 0.264 |
| Left brain | 0.180 | 0.227 | 0.803 |

The experimental results of Table 1 and Table 2 show that the boron-containing drug prepared in this invention is highly uptaken at tumor sites such as subcutaneous tumors and orthotopic brain gliomas, and can pass through the blood-brain barrier to enrich in brain gliomas. The T/N ratio is at least 7:1 (Table 2) and can reach up to 70:1 (Table 1), solving the defect of common nanodrugs that cannot pass through the blood-brain barrier.

### Examples 6-12: Preparation of Other Boron-Containing Drugs

The preparation of Examples 6-12 refers to Example 1, and the amounts of each component in Table 3 are expressed in weight parts.

**Table 3: Components and Properties of Boron-Containing Drugs of Examples 6-12**

| Examples | Dopamine and its derivatives and amounts | Boron-containing compounds and amounts | Boron contents | Average particle sizes |
|---|---|---|---|---|
| Example 6 | Dopamine, 1 part | BPA, 1 part | 0.71% | 137 nm |
| Example 7 | Dopamine, 10 parts | BPA, 1 part | 0.13% | 300 nm |
| Example 8 | Dopamine, 4 parts | Boron 10 acid, 1 part | 0.84% | 226 nm |
| Example 9 | Dopamine, 5 parts | 5-amino-2,3-difluorophenyl boric acid, 1 part | 0.22% | 600 nm |
| Example 10 | Dopamine, 3 parts | 3-amino-4-methylphenyl boric acid, 1 part | 0.15% | 451 nm |
| Example 11 | L-methyldopa, 2.5 parts | BPA, 1 part | 0.37% | 480 nm |
| Example 12 | 5-hydroxydopamine, 5 parts | BPA, 1 part | 0.24% | 120 nm |

At a dosage of 150mg/kg, the boron-containing drug prepared in Examples 6-12 showed a T/N ratio ranging from 5:1 to 70:1 in the distribution experiment of a mouse subcutaneous brain glioma model. The SEM image of the boron-containing drug of Example 8 is shown in Fig. 7.

### Example 13: Photothermal Experiment of Boron-Containing Drug

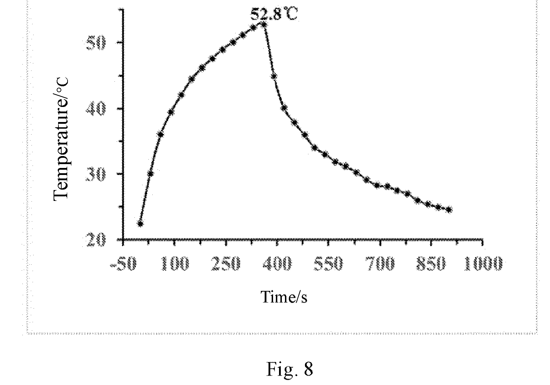

A semiconductor laser subsystem was used to emit an 808nm near-infrared laser, and the spot area irradiated by the laser was set to be 0.6 cm². An aqueous solution of 0.1mg/mL boron-containing drug of Example 1 was added to a quartz cuvette, and irradiated with a near-infrared excitation light source (wavelength of 808nm) at a power density of 1.5W/cm² for 15 minutes. The temperature of the solution was measured every 30 seconds with a thermometer. The specific results are shown in Fig. 8. The photothermal conversion efficiency of the boron-containing drug was calculated to be 39.46%.

### Example 14: Photothermal Therapy of Subcutaneous Brain Glioma in Mice with Boron-Containing Drug

Normal saline and 150mg/kg of the boron-containing drug of Example 1 were injected into tumor-bearing mice via the tail vein, respectively. The mice were irradiated with a near-infrared excitation light source (wavelength of 808nm) for 30 minutes. The volume of the subcutaneous brain gliomas was measured on days 3, 5, and 10. The specific results are shown in Table 4.

**Table 4: Volume of Subcutaneous brain Glioma in Mice**

| Time | Normal saline group | Boron-containing drug group |
|---|---|---|
| Day 3 | 107 mm³ | 110 mm³ |
| Day 5 | 467 mm³ | 98 mm³ |
| Day 10 | 1876 mm³ | 73 mm³ |

Finally, it should be noted that the above examples are provided to illustrate the technical solutions of the present invention and not to limit them. Although the present invention has been described in detail with reference to the preceding examples, those of ordinary skill in the art should understand that they can still make modifications to the technical solutions recorded in the preceding examples, or make equivalent replacements for some or all of the technical features. Such modifications or replacements do not cause the essence of the corresponding technical solutions to depart from the scope of the technical solutions of the examples of the present invention.

## Claims

1. A boron-containing drug which is obtained by polymerizing dopamine and its derivative with a boron-containing compound.

2. The boron-containing drug according to claim 1, wherein the boron-containing compound includes, but is not limited to, any one or more of boric acid, phenylalanine boric acid and its derivative, proline boric acid, aspartic acid boric acid, tyrosine boric acid, cysteine boric acid, methionine boric acid, serine boric acid, 5-amino-2,3-difluorophenyl boric acid, 3-amino-5-cyanophenyl boric acid, 3-amino-4-methylphenyl boric acid, 3-amino-phenylboric acid hydrochloride, and 4-carbamoyl phenylboric acid.

3. The boron-containing drug according to claim 2, wherein the boron-containing compound is phenylalanine boric acid and its derivative.

4. The boron-containing drug according to claim 1, wherein the dopamine and its derivative comprise any one or more of dopamine, 4-(2-aminopropyl)phenyl-1,2-diol, norepinephrine, L-methyldopa, droxidopa, and 5-hydroxydopamine;
preferably, the dopamine and its derivative are dopamine, L-methyldopa, or 5-hydroxydopamine.

5. The boron-containing drug according to claim 1, wherein the weight ratio of the dopamine and its derivative to the boron-containing compound is (1-10) : 1.

6. The boron-containing drug according to claim 5, wherein the weight ratio of the dopamine and its derivative to the boron-containing compound is (3-4) : 1.

7. The boron-containing drug according to claim 1, wherein the boron content in the boron-containing drug is 0.1% to 1.0%.

8. The boron-containing drug according to any one of claims 1-7, wherein an indication for the boron-containing drug comprises head and neck tumors, lung cancer, peritoneal cancer, hepatocellular cancer, gastric cancer, melanoma, pancreatic cancer, brain glioma, cervical cancer, ovarian cancer, liver cancer, bladder cancer, breast cancer, colon cancer, colorectal cancer, uterine cancer, salivary gland cancer, kidney cancer, prostate cancer, vulvar cancer, thyroid cancer, B cell lymphoma or leukemia.

9. The boron-containing drug according to claim 8, wherein the indication for the boron-containing drug is head and neck tumors, melanoma or brain glioma.

10. A method for preparing a boron-containing drug according to any one of claims 1-9, comprising steps of: mixing and reacting an aqueous solution of dopamine and its derivative with an aqueous solution of a boron-containing compound, separating a solid from a liquid to obtain the solid, and washing and freeze-drying the solid.

11. The method according to claim 10, wherein the mixing and reacting is performed at pH from 6 to 14.

12. The method according to claim 11, wherein a time of the mixing and reacting is 0.5 hour to 48 hours, and a stirring speed during the reacting is over 200 RPM.
